# EUROPEAN PATENT APPLICATION

(11) **EP 2 397 038 A1**
(43) Date of publication of application: **21.12.2011**
(21) Application number: 10380082.7
(22) Date of filing: 21.06.2010
(51) Int. Cl.: A23L 1/29, A23L 1/305

(54) **Early programming of brain function through soy protein feeding**

(71) Applicant: Abbott Laboratories, Columbus OH 43219 (US)
(72) Inventor: Rueda Cabrera, Ricardo, 18008 Granada (ES); Barranco Perez, Alejandro, 18195 Cullar-Vega (Granada) (ES); Ramirez Gonzalez, Maria, 18003 Granada (ES); Jesus Martin, Maria, 18006 Granada (ES)
(74) Representative: Ungria Lopez, Javier

(57) **Abstract**

Disclosed are methods for improving memory and/or cognition in an infant by administering soy protein to the infant's mother during pregnancy, and further administering soy protein to the infant after delivery. The soy protein may be administered to the infant's mother during lactation if the infant is breastfed and/or may be directly administered, for example, by administering the infant an infant and/or nutritional formula comprising soy protein. Also disclosed are compositions comprising soy protein that may be used in the methods.

## Description

### BACKGROUND OF THE DISCLOSURE

The present disclosure relates to methods for improving memory and/or cognition of an infant that include administering soy protein to the infant's mother during pregnancy and breastfeeding, and further administering soy protein to the infant when mother's milk is no longer available, for example, by administering the infant an infant and/or nutritional formula comprising soy protein. Also disclosed are compositions comprising soy protein that may be used in the methods.

Advantages have been recognized to inclusion of soy protein in the diet. For instance, various studies have suggested that soy protein may play a role in the prevention of menopausal symptoms, osteoporosis, certain types of cancer, and heart disease. It has also been suggested that soy protein may help reduce levels of low density lipoprotein (LDL) cholesterol.

Soy-based infant formulas are also well known and readily available from a number of commercial sources, including Similac® Isomil® Advance® Infant Formula available from Abbott Nutrition, Columbus, Ohio. These soy-based formulas are prepared especially for infants with feeding problems such as fussiness, gas, and spit-up, as well as for infants whose parents choose a non-milk-based formula as a first feeding or as a supplement to breastfeeding. These soy-based formulas are especially helpful for those infants with allergies or sensitivities to cow's-milk protein, and for those infants with disorders for which lactose from cow's milk should be avoided.

It has now unexpectedly been discovered that an improvement in memory and/or cognition in an infant can be achieved by administering soy protein during early brain development. Specifically, the soy protein may be administered to the infant's mother during gestation and breastfeeding, and to the infant after delivery, for example, by administering the infant an infant and/or nutritional formula comprising soy protein as an alternative to or in addition to breastfeeding. By administering soy protein to an infant during early brain development, the memory and/or cognition of the infant is improved.

### SUMMARY OF THE DISCLOSURE

The present disclosure is directed to a method of improving memory in an infant. The method comprises administering to a pregnant woman soy protein for at least about three months prior to delivery of the infant; and after delivery, administering soy protein to the infant for at least about 4 months, or 5 months, or six months, or seven months, or eight months or nine months or even 12 months.

In another aspect, the present disclosure is directed to a method of improving memory in an infant. The method comprises administering to a pregnant woman soy protein for at least about three months prior to delivery of the infant; after delivery, administering soy protein to the woman during breastfeeding of the infant until weaning; and after weaning, administering an infant formula comprising soy protein to the infant until the infant has reached at least about 4 months, or even 5 months, or even 6 months, or even 7 months, or even 8 months, or even 9 months or even 12 months of age.

In still another aspect, the present disclosure is directed to a method of improving cognition in an infant. The method comprises administering to a pregnant woman soy protein for at least about three months prior to delivery of the infant; and after delivery, administering soy protein to the infant for at least about 4, or even 5, or even 6, or even 7, or even 9 or even 9, or even 12 months.

It has been unexpectedly discovered that an improvement in memory and/or cognition in an infant can be achieved by administering soy protein to the infant and/or the infant's mother during early brain development. Specifically, the soy protein may be administered to the infant's mother during pregnancy (i.e., gestation) and breastfeeding, and may also be directly administered to the infant after delivery, for example, by administering an infant and/or nutritional formula comprising soy protein to the infant as an alternative to or in addition to breastfeeding. Following delivery, the soy protein may be administered to the infant until the infant reaches at least about 12 months of age, or until the infant reaches from about 1 year of age to about 5 years of age.

### BRIEF DESCRIPTION OF THE DRAWINGS

**Figure 1** is a graph showing the mean (±set) of platform reaching latency (seconds) for the Group 1 and Group 2 rats for the six training blocks in the Morris hidden platform task, as discussed in Example 7.
**Figure 2** is a graph showing the mean (±SEM) of swimming speed of the Group 1 and Group 2 rats for the six training blocks in the Morris hidden platform task, as discussed in Example 7.
**Figure 3** is a graph showing the mean (±set) path length of the Group 1 and Group 2 rats for the six training blocks in the Morris hidden platform task, as discussed in Example 7.
**Figure 4** is a chart showing the mean (±set) time spent in each quadrant of the pool for the Group 1 and Group 2 rats during the probe trial 24 hours after training in the Morris hidden platform task, as discussed in Example 7.
**Figure 5** is a chart showing the mean (±SEM) first entrance latencies in the target quadrant of the pool for the Group 1 and Group 2 rats during the probe trial 24 hours after training in the Morris hidden platform task, as discussed in Example 7.
**Figure 6** is a chart showing the mean (±set) time spent in the target quadrant for the Group 1 and Group 2 rats during the probe trial 48 hours after training in the Morris hidden platform task, as discussed in Example 7.

### DETAILED DESCRIPTION OF THE DISCLOSURE

The present disclosure is directed to methods for improving memory and/or cognition in infants. The methods include administering soy protein to the infant's mother during pregnancy (i.e., gestation), and further administering soy protein to the infant after delivery for a certain period of time. For example, following delivery, the infant's mother may be administered the soy protein during lactation if the infant is breastfed and/or the soy protein may be directly administered to the infant, for example, by administering the infant an infant and/or nutritional formula comprising soy protein. Also disclosed are nutritional formulas or other compositions comprising soy protein that may be used in the methods of the present disclosure. These and other essential or optional elements or limitations of the soy protein containing compositions and methods of the present disclosure are described in detail hereafter.

The term "infant" as used herein, unless otherwise specified, refers to children not more than about one year of age, and includes infants from 0 to about 4 months of age, infants from about 4 to about 8 months of age, infants from about 8 to about 12 months of age, low birth weight infants at less than 2,500 grams at birth, and preterm infants born at less than about 37 weeks gestational age, typically from about 26 weeks to about 34 weeks gestational age. The term "child" or "children" as used herein refers to children not more than 12 years of age, and includes children from about 12 months to about 12 years of age. The term "adult" as used herein refers to adults and children about 12 years and older.

The term "infant formula" as used herein, unless otherwise specified, refers to a nutritional formula designed for infants that contains sufficient nutrients such as proteins, carbohydrates, lipids, vitamins, and minerals to potentially serve as a supplemental, primary, or sole source of nutrition.

The term "nutritional formula" as used herein, unless otherwise specified, refers to a nutritional composition designed for infants, toddlers, children, adults, or combinations thereof, that contains sufficient nutrients such as proteins, carbohydrates, lipids, vitamins, minerals, and electrolytes to potentially serve as a supplemental, primary, or sole source of nutrition.

The term "ready-to-feed" as used herein, unless otherwise specified, refers to nutritional formulas in liquid form suitable for administration, including reconstituted powders, diluted concentrates, and manufactured liquids.

All percentages, parts and ratios as used herein, are by weight of the total composition, unless otherwise specified. All such weights as they pertain to listed ingredients are based on the active level and, therefore, do not include solvents or byproducts that may be included in commercially available materials, unless otherwise specified.

Numerical ranges as used herein are intended to include every number and subset of numbers within that range, whether specifically disclosed or not. Further, these numerical ranges should be construed as providing support for a claim directed to any number or subset of numbers in that range. For example, a disclosure of from 1 to 10 should be construed as supporting a range of from 2 to 8, from 3 to 7, from 5 to 6, from 1 to 9, from 3.6 to 4.6, from 3.5 to 9.9, and so forth.

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The compositions of the present disclosure may also be substantially free of any optional or selected essential ingredient or feature described herein, provided that the remaining composition still contains all of the required ingredients or features as described herein. In this context, and unless otherwise specified, the term "substantially free" means that the selected composition contains less than a functional amount of the optional ingredient, typically less than 0.1 % by weight, and also including zero percent by weight of such optional or selected essential ingredient.

The compositions, nutritional formulas, infant formulas, and corresponding methods of use of the present disclosure can comprise, consist of, or consist essentially of the essential elements and limitations of the disclosure as described herein, as well as any additional or optional ingredients, components, or limitations described herein or otherwise useful in nutritional formula applications.

### Soy Protein

It has surprisingly been discovered that an improvement in memory and/or cognition in an infant can be achieved by administering soy protein to a pregnant woman for at least about three months prior to delivery of the infant and, after delivery, administering soy protein to the infant for at least about 4 months, or even 5 months, or even 6 months, or even 7 months, or even 8 months, or even 9 months or even 12 months. After delivery, soy protein may be administered to the infant's mother during breastfeeding and/or directly administered to the infant, for example, by administering a soy protein-containing composition, such as an infant or nutritional formula comprising soy protein, to the infant. By administering soy protein to an infant during early brain development, for example, during gestation and within the first 12 months following delivery, the memory and/or cognition of the infant is improved.

When the infant is directly administered an infant or nutritional formula comprising soy protein, the infant will preferably receive at least about 4 g/day of soy protein, more typically from about 4 g/day to about 40 g/day of soy protein, and preferably from about 6 g/day to about 18 g/day of soy protein.

The soy protein is administered to the infant's mother during pregnancy and, in some embodiments, during breastfeeding of the infant, in the form of a soy protein-containing composition (also referred to herein as a soy protein composition), for example, a nutritional composition. Preferably, the soy protein composition will provide the pregnant or breastfeeding woman at least about 3 g/day of soy protein, more typically from about 3 g/day to about 54 g/day of soy protein, and more preferably from about 8 g/day to about 18 g/day of soy protein. Administering these amounts of soy protein to the pregnant or breastfeeding woman results in an improvement in memory and/or cognition in the infant.

The soy protein-containing compositions used in the methods of the present disclosure may comprise any suitable source of soy protein. Examples of suitable sources of soy protein include, but are not limited to, soy flakes, soy protein isolates, soy protein concentrate, hydrolyzed soy protein, soy flour, soy protein fiber, soy whey, or any other protein or protein source derived from soy or mixtures thereof. The soy protein suitable for use in the soy protein compositions of the present disclosure includes extensively hydrolyzed, partially hydrolyzed or non-hydrolyzed soy proteins or protein sources.

Commercial sources of soy protein are also well known in the nutrition art, some non-limiting examples of which include soy protein isolates distributed by The Solae Company under the trade designation Soy Protein Isolate EXP-H0118,EXP-E-0101, SUPRO PLUS 675, SUPRO 670, SUPRO 710, SUPRO 620, SUPRO 500E, SUPRO 630, and SUPRO EX33; and PROFAM 931, PROFAM 873, and PROFAM 891, available from Archer Daniels Midland (Decatur, Ill.). Examples of such soy protein isolates and methods of producing them are described in U.S. Patent No. 7,323,200, herein incorporated by reference in its entirety.

A variety of soy protein-containing foods are also currently available. The nutrient content of some commonly available soy foods and soy ingredients are listed in Table 1 below.

**Table 1: Nutrient Content of Different Soy Foods and Ingredients***

| **Soy Food** | **Calories (kcal)** | **Protein (g)** |
|---|---|---|
| Miso (1 oz) | 35 | 2 |
| Soybeans, cooked (1/2 cup) | 149 | 14 |
| Soy flour (3½ cup) | 441 | 35 |
| Soymilk (1 cup) | 140 | 10 |
| Soy milk, low-fat (1 cup) | 120 | 8 |
| Soy nuts, dry roasted (1/2 cup) | 387 | 34 |
| Soy protein isolate (1 oz) | 94 | 25 |
| Tempen (4 oz) | 204 | 17 |
| Tofu, low-fat (3 oz) | 35 | 6 |
| Tofu, extra-firm (3 oz) | 60 | 6 |
| Textured vegetable protein (1/4 cup, dry) | 59 | 11 |
| Ensure® (8 fl oz) | 250 | 8.8 |
| GeniSoy™ (35 gm) | 130 | 14 |

| | | |
|---|---|---|
| *Table adapted from us Department of Agriculture Handbook 8. | | |

### Nutrients

The soy protein used in the methods of the present disclosure can be incorporated into any food or beverage that can be consumed by human infants or adults or animals. Thus, in one aspect, the soy protein composition used in the methods of the present disclosure is formulated as a nutritional or infant formula. The nutritional or infant formulas of the present disclosure may comprise sufficient types and amounts of nutrients to meet the targeted dietary needs of the intended user. These nutritional or infant formulas may therefore comprise protein, carbohydrate, and a lipid component (all either organic or non-organic) in addition to the soy protein discussed above. The nutritional or infant formulas may also include vitamins, minerals, or other ingredients suitable for use in nutritional formulas.

When the nutritional formula is an adult formula, for example for feeding to a pregnant or breastfeeding woman, the protein component may comprise from about 10% to about 80% of the total caloric content of said nutritional formula; the carbohydrate component may comprise from about 10% to about 70% of the caloric content of said nutritional formula; and the lipid component may comprise from about 5% to about 50% of the total caloric content of said nutritional formula. At least about 30 % by weight of the protein component may comprise soy protein, more typically at least about 50% by weight, and up to 100% of the protein component may comprise soy protein. The nutritional formula may be in liquid or powder form. These ranges are provided as examples only, and are not intended to be limiting.

When the nutritional formula is a non-adult formula, such as an infant formula, the non-adult formula includes those embodiments in which the protein component may comprise from about 7.5% to about 25% of the caloric content of the nutritional formula; the carbohydrate component may comprise from about 35% to about 50% of the total caloric content of the nutritional formula; and the lipid component may comprise from about 30% to about 60% of the total caloric content of the nutritional formula. At least about 30% by weight of the protein component may comprise soy protein, more typically at least about 50% by weight, and up to 100% of the protein component may be soy protein. These ranges are provided as examples only, and are not intended to be limiting. Additional suitable ranges are noted in the following Table.

| Nutrient* | 1^{st} Embodiment | 2^{nd} Embodiment | 3^{rd} Embodiment |
|---|---|---|---|
| Carbohydrates: % total calories | 20-85 | 30-60 | 35-55 |
| Lipid: % total calories | 5-70 | 20-60 | 25-50 |
| Protein: % total calories | 2-75 | 5-50 | 7-40 |

| | | | |
|---|---|---|---|
| *all numerical values preceded by the term "about" | | | |

Many different sources and types of carbohydrates, lipids, proteins, minerals and vitamins are known and can be used in the nutritional formulas of the present disclosure,provided that such nutrients are compatible with the added ingredients in the selected formula, are safe for their intended use, and do not otherwise unduly impair product performance.

Carbohydrates suitable for use in the nutritional formulas of the present disclosure can be simple, complex, or variations or combinations thereof. Non-limiting examples of suitable carbohydrates include hydrolyzed, intact, naturally and/or chemically modified cornstarch, maltodextrin, glucose polymers, sucrose, corn syrup, corn syrup solids, rice or potato derived carbohydrate, glucose, fructose, lactose, high fructose corn syrup and indigestible oligosaccharides such as fructooligosaccharides (FOS), and combinations thereof.

Non-limiting examples of proteins suitable for use in the nutritional formulas, in addition to the soy protein discussed above, include extensively hydrolyzed, partially hydrolyzed or non-hydrolyzed proteins or protein sources, and can be derived from any known or otherwise suitable source such as milk (e.g., casein, whey), animal (e.g., meat, fish), cereal (e.g., rice, corn), vegetable (e.g., soy), or combinations thereof. The proteins for use herein can also include, or be entirely or partially replaced by, free amino acids known for use in nutritional formulas, non-limiting examples of which include tryptophan, glutamine, tyrosine, methionine, cysteine, arginine, and combinations thereof. Other (non-protein) amino acids typically added to nutritional formulas include carnitine and taurine. In some cases, the D-forms of the amino acids are considered as nutritionally equivalent to the L-forms, and isomer mixtures are used to lower cost (for example, D,L-methionine).

Non-limiting examples of lipids suitable for use in the nutritional formulas include coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, marine oils, cottonseed oils, long-chain polyunsaturated fatty acids such as arachidonic acid (ARA), docosahexaenoic acid (DHA), and eicosapentaenoic acid (EPA), and combinations thereof.

In addition to these food grade oils, structured lipids may be incorporated into the nutritional formulas if desired. Structured lipids are known in the art. Structured lipids are predominantly triacylglycerols containing mixtures of medium and long chain fatty acids on the same glycerol nucleus. Structured lipids are also described in U.S. Pat. No. 6,160,007, which is also incorporated herein by reference.

The nutritional formulas of the present disclosure may further comprise any of a variety of vitamins in addition to the components described above. Non-limiting examples of vitamins include vitamin A, vitamin D, vitamin E, vitamin K, thiamine, riboflavin, pyridoxine, vitamin B 12, niacin, folic acid, pantothenic acid, biotin, vitamin C, choline, chromium, carnitine, inositol, salts and derivatives thereof, and combinations thereof.

The nutritional formulas may further comprise any of a variety of minerals, non-limiting examples of which include calcium, phosphorus, magnesium, iron, zinc, manganese, copper, iodine, sodium, potassium, chloride, and combinations thereof.

The infant formulas of the present disclosure preferably comprise nutrients in accordance with the relevant infant formula guidelines for the targeted consumer or user population, as example of which would be the Infant Formula Act, 21 U.S.C. Section 350(a).

The nutritional formulas of the present disclosure include those embodiments containing the carbohydrate, lipid, and protein concentrations described in Table 2 (Nutritional Formula Macronutrients).

**Table 2***

| Nutrient | Embodiment | g/100 kcal | g/100 g total solids | g/L (as fed) |
|---|---|---|---|---|
| Carbohydrate | 1^{st} Embodiment | 8-16 | 30-90 | 54-108 |
| | 2^{nd} Embodiment | 9-13 | 45-60 | 57-79 |
| | 3^{rd} Embodiment | 15-19 | 63-81 | 157-203 |
| Lipid | 1^{st} Embodiment | 3-8 | 15-42 | 20-54 |
| | 2^{nd} Embodiment | 4-6.6 | 20-30 | 27-45 |
| | 3^{rd} Embodiment | 2-5 | 8-21 | 20-53 |
| Protein | 1^{st} Embodiment | 1-3.9 | 8-20.5 | 7-24 |
| | 2^{nd} Embodiment | 1.5-3.4 | 10-17 | 10-23 |
| | 3^{rd} Embodiment | 3.5-6.0 | 14.8-25.3 | 37-63 |

| | | | | |
|---|---|---|---|---|
| *all numerical values preceded by the term "about" | | | | |

The nutritional formulas of the present disclosure include those embodiments that comprise per 100 kcal of reconstituted formula one or more of the following: vitamin A (from about 250 to about 1250 IU), vitamin D (from about 40 to about 150 IU), vitamin K (greater than about 4 mcg), vitamin E (at least about 0.3 IU), vitamin C (at least about 8 mg), thiamine (at least about 8 mcg), vitamin B12 (at least about 0.15 mcg), niacin (at least about 250 mcg), folic acid (at least about 4 mcg), pantothenic acid (at least about 300 mcg), biotin (at least about 1.5 mcg), choline (at least about 7 mg), and inositol (at least about 4 mg).

The nutritional formulas of the present disclosure include those embodiments that comprise per 100 kcal of reconstituted formula one or more of the following: calcium (at least about 50 mg), phosphorus (at least about 25 mg), magnesium (at least about 6 mg), iron (at least about 0.15 mg), iodine (at least about 5 mcg), zinc (at least about 0.5 mg), copper (at least about 60 mcg), manganese (at least about 5 mcg), sodium (from about 20 to about 60 mg), potassium (from about 80 to about 200 mg), and chloride (from about 55 to about 150 mg).

### Optional Ingredients

The soy protein compositions of the present disclosure may further comprise other optional components that may modify the physical, chemical, aesthetic or processing characteristics of the compositions or serve as pharmaceutical or additional nutritional components when used in the targeted population. Many such optional ingredients are known or other suitable for use in food and nutritional products, including infant formulas, and may also be used in the soy protein compositions of the present disclosure, provided that such optional materials are compatible with the essential materials described herein, are safe for their intended use, and do not otherwise unduly impair product performance.

Non-limiting examples of such optional ingredients include preservatives, antioxidants, emulsifying agents, buffers, colorants, flavors, nucleotides, and nucleosides, additional probiotics, additional prebiotics, lactoferrin, and related derivatives, thickening agents and stabilizers, and so forth.

### Product Form

The soy protein compositions of the present disclosure may be prepared as any product form suitable for administration to humans, including liquid or powdered complete nutritionals, liquid or powdered supplements (such as a supplement that can be mixed with a drink), reconstitutable powders, ready-to-feed liquids, solid matrix nutritionals, liquid or powdered human milk fortifiers, beverages, nutritional bites (e.g., plurality of smaller nutritional product dosage forms in a single package), nutritional bars (snack or meal replacement), and dilutable liquid concentrates, among others, which product forms are all well known in the nutritional formula arts.

The soy protein compositions may be formulated to include only soy protein or a source thereof as described herein, or alternately, may be combined with optional ingredients to form a number of different product forms. The soy protein compositions of the present disclosure are preferably formulated as a nutritional composition, which may include a liquid or powdered nutritional or infant formula, a liquid or powdered human milk fortifier, or solid matrix nutritionals, among others.

The soy protein compositions of the present invention, when formulated as a nutritional composition, may potentially provide either a sole, primary, or supplemental source of nutrition to an individual. In this context, a sole source of nutrition is one that can be administered once or multiple times each day to potentially provide an individual with all or substantially all their fat, protein, carbohydrate, mineral, and vitamin needs per day or during the intended period of administration. A supplemental source of nutrition is defined herein as a dietary source that does not provide an individual with a potentially sole source of nutrition. The soy protein compositions of the present disclosure may have any caloric density suitable for the targeted or intended patient population, i.e., pregnant or breastfeeding women or infants, or provide such a density upon reconstitution of a powder embodiment or upon dilution of a liquid concentrate embodiment. For example, most common caloric densities for the infant formula embodiments of the present disclosure are generally at least about 19 kcal/fl oz (660 kcal/liter), more typically from about 20 kcal/fl oz (675-680 kcal/liter) to about 25 kcal/fl oz (820 kcal/liter), even more typically from about 20 kcal/fl oz (675-680 kcal/liter) to about 24 kcal/fl oz (800-810 kcal/liter). Generally, the 22-24 kcal/fl oz formulas are more commonly used in preterm or low birth weight infants, and the 20-21 kcal/fl oz (675-680 to 700 kcal/liter) formulas are more often used in term infants. Non-infant and adult nutritional formulas may have any caloric density suitable for the targeted or intended population.

In one embodiment, the soy protein composition is formulated for direct administration to an infant and may be, for example, an infant formula. Examples of components suitable for inclusion in an infant formula are described above. Soy protein based infant formulas are also readily available from a number of commercial sources, including Similac® Isomil® Advance® Infant Formula available from Abbott Nutrition, Columbus, Ohio. Examples of suitable soy-based infant formulas are also described in U.S. Patent No. 7,323,200.

In some embodiments, the soy protein composition is administered to the infant until the infant reaches from about 1 year of age to about 5 years of age. In these embodiments, as the infant gets older, soy protein based nutritional formulas suitable for young children may be administered. For example, a suitable nutritional formula, such as PediaSure®, available from Abbott Nutrition, Columbus, Ohio, may be re-formulated to include soy protein and administered to the child.

In another embodiment, the soy protein composition may be a soy protein based liquid or powdered human milk fortifier which, when combined with human milk, may be administered to the infant to provide soy protein to the infant in accordance with the methods of the present disclosure.

The soy protein composition may also be formulated for administration to a pregnant or breastfeeding woman in accordance with the methods of the present disclosure. In one particular embodiment, the soy protein composition is an adult nutritional formula. Examples of suitable components for inclusion in a nutritional formula are described above. Commercially available soy protein compositions suitable for consumption by a pregnant or breastfeeding woman in accordance with the methods of the present disclosure include the liquid nutritional, Ensure® (Abbott Nutrition, Columbus, Ohio), which is a good tasting, shelf stable, ready to drink complete nutritional with a protein system that contains soy protein isolate. Other examples of suitable soy protein-containing compositions that may be administered to a pregnant or lactating woman in accordance with the methods of the present disclosure are described in, for example, U.S. 6,372,782; U.S. 6,811,804; and U.S. 7,090,862, all herein incorporated by reference in their entirety. Other nutritional products intended for pregnant or lactating women, such as Similac Mom®, can also be used, provided that they are formulated to include soy protein. For instance, Similac Mom® does not contain soy protein, but one skilled in the art could readily reformulate this product to include soy protein in accordance with the methods of the present disclosure.

For nutritional powder embodiments of the present disclosure, whether formulated for administration to the infant or the pregnant or breastfeeding woman, such powders are typically in the form of flowable or substantially flowable particulate compositions, or at least particulate compositions that can be easily scooped and measured with a spoon or similar other device, wherein the compositions can easily be reconstituted by the intended user with a suitable aqueous fluid, typically water, to form a liquid nutritional formula for immediate oral or enteral use. In this context, "immediate" use generally means within about 48 hours, most typically within about 24 hours, preferably right after reconstitution. These powder embodiments include spray dried, agglomerated, dry mixed or other known or otherwise effective particulate form. The quantity of a nutritional powder required to produce a volume suitable for one serving may vary.

The nutritional formulas of the present disclosure may be packaged and sealed in single or multi-use containers, and then stored under ambient conditions for up to about 36 months or longer, more typically from about 12 to about 24 months. For multi-use containers, these packages can be opened and then covered for repeated use by the ultimate user, provided that the covered package is then stored under ambient conditions (e.g., avoid extreme temperatures) and the contents used within about one month or so.

In one particular embodiment, soy protein may be incorporated into a solid matrix nutritional and administered to a pregnant or breastfeeding woman in accordance with the methods of the present disclosure. Such a solid matrix nutritional comprises a soy protein source, optionally an additional protein source, a fat source, a carbohydrate source, vitamins, and minerals in amounts sufficient to supplement a pregnant or breastfeeding woman's normal diet. Such amounts are well known by those skilled in the art and can be readily calculated when preparing such products, and include those set forth above for nutritional formulas.

Typically, one 60 gm bar (230 calories) of the nutritional for a pregnant woman will comprise 6 gm of total fat (24% of calories), 9 gm of protein (16% of calories), 35 gm of carbohydrates (60% of calories) and suitable amounts of vitamins and minerals.

Typically, one 50 gm bar (220 calories) for the breastfeeding woman will comprise 6 gm of total fat (24% of calories), 8 gm of protein (14% of calories), 34 gm of carbohydrates (62% of calories) and suitable amount of vitamins and minerals.

The solid matrix nutritional may also desirably include a coating, flavoring and/or color to provide the nutritional compositions with an appealing appearance and an acceptable taste for oral consumption. For example, the nutritional bar of Example 6 is coated with sugar free white confectionery coating. Examples of suitable coatings typically include compounded confectionery coating, milk chocolate coating, glazes, shellac, sugar free compounded confectionery coating, sugar free glazes and sugar free shellac. Examples of useful flavorings for the solid matrix nutritional typically include, for example, chocolate, butter pecan, strawberry, cherry, orange, peanut butter, graham and lemon.

The solid matrix nutritional may also desirably include ingredients, which add texture to enhance the mouth feel of the solid matrix nutritional. For example, crisp rice was added at about 6.4 wt/wt % of the nutritional bar in Example 6. Examples of other suitable ingredients, which can add texture typically, include nuts, soy nuggets, toasted oats, and fruit pieces.

Other specific examples of soy protein composition product forms suitable for administration to a pregnant or breastfeeding woman in accordance with the methods of the present disclosure include most any conventional or otherwise known food product form, such as confectionary products, cereals, food condiments (e.g., spreads, powders, sauces, jams, jelly, coffee creamer or sweetener), pasta, baking or cooking materials (e.g., flour, fats or oils, butter or margarine, breading or baking mixes), salted or seasoned snacks (e.g., extruded, baked, fried), beverages (e.g., coffee, juice, carbonated beverage, non-carbonated beverage, tea, ice-cream based drinks), snack or meal replacement bars (e.g., Ensure™ bars, ZonePerfect bars™, Glucerna™ bars), smoothies, breakfast cereals, cheeses, gummie products, salted or unsalted crisp snacks (e.g., chips, crackers, pretzels), dips, baked goods (e.g., cookies, cakes, pies, pastries, bread, bagels, croutons, dressings, dry mixes (e.g., mixes for muffins, cookies, waffles, pancakes, beverages), frozen desserts (e.g., ice cream, popsicles, fudge bars, crushed ice, frozen yogurt), pasta, processed meats (e.g., corn dogs, hamburgers, hotdogs, sausage, pepperoni), pizza, pudding, flavored or unflavored gelatin, refrigerated dough (e.g., cookies, bread, brownies), milk or soy-based smoothies, yogurt or yogurt-based drinks, frozen yogurt, soy milk, soups, vegetable-based burgers, and popcorn-based snacks.

### Methods of Manufacture

The soy protein compositions of the present disclosure may be prepared by any known or otherwise effective technique suitable for making and formulating a nutritional formula or similar other product, variations of which may depend upon variables such as the selected product form, such as nutritional liquids or nutritional bars; ingredient combination; packaging and container selection; and so forth, for the desired composition. Such techniques and variations for any given composition are easily determined and applied by one of ordinary skill in the nutritional formulation or manufacturing arts.

The nutritional formulas of the present disclosure, including some of the exemplified formulas described hereinafter, can therefore be prepared by any of a variety of known or otherwise effective formulation or manufacturing methods. These methods most typically involve the initial formation of an aqueous slurry containing carbohydrates, proteins, lipids, stabilizers or other formulation aids, vitamins, minerals, or combinations thereof. The slurry is emulsified, pasteurized, homogenized, and cooled. Various other solutions, mixtures, or other materials may be added to the resulting emulsion before, during, or after further processing. This emulsion can then be further diluted, heat-treated, and packaged to form a ready-to-feed or concentrated liquid, or it can be heat-treated and subsequently processed and packaged as a reconstitutable powder, e.g., spray dried, dry mixed, agglomerated.

Other suitable methods for making nutritional formulas are described, for example, in U.S. Pat. No. 6,365,218 (Borschel, et al.), U.S Patent 6,589,576 (Borschel, et al.), U.S. Pat. No. 6,306,908 (Carlson, et al.), U.S. Patent Application 20030118703 A1 (Nguyen, et al.), which descriptions are incorporated herein by reference.

The solid matrix nutritional compositions may be manufactured using cold extrusion technology as is known in the art. To prepare such compositions, typically all of the powdered components will be dry blended together. Such constituents typically include the proteins, vitamin premixes, certain carbohydrates, and so forth. The fat soluble components are then blended together and mixed with the powdered premix above. Finally any liquid components are then mixed into the composition, forming a plastic like composition or dough.

The process above is intended to give a plastic mass, which can then be shaped, without further physical or chemical changes occurring, by the procedure known as cold forming or extrusion. In this process, the plastic mass is forced at relatively low pressure through a die, which confers the desired shape, and the resultant exudate is then cut off at an appropriate position to give products of the desired weight.

The mass may, for example, be forced through a die of small cross-section to form a ribbon, which is carried on a belt moving at a predetermined speed under a guillotine type cutter which operates at regular intervals. The cutter, in this case, generally consists of a sharpened blade so adjusted that it cuts through the ribbon but not the underlying belt, but may also consist of a wire. In both cases, the principle is the same; the cutting process occurs at intervals that permit the moving ribbon to be cut into pieces of equivalent weight and dimensions. Generally, this is achieved by timing the cutting strokes and maintaining belt speed at an appropriate level, but there also exist computer controlled versions of this mechanism which offer greater versatility. Alternatively, the mass may be forced through a die of large cross-section and then cut at die level into slices by an oscillating knife or wire, which drop onto a moving belt and are thus transported away. The mass may also be extruded as a sheet, which is then cut with a stamp type cutter into shapes that are appropriate, such as a cookie type cutter. Finally, the mass may also be forced into chambers on a rotary die equipped with an eccentric cam that forces the thus-formed material out of the chamber at a certain point in a rotation of the cylindrical die.

After shaping, the formed product is moved by a transfer belt or other type of material conveyor to an area where it may be further processed or simply packaged. In general, a nutritional bar of the type described can be enrobed (coated) in a material that may be chocolate, a compound chocolate coating, or some other type of coating material. The coating material typically consists of a fat that is solid at room temperature, but that is liquid at temperature in excess of e.g. 31°C, together with other materials that confer the organoleptic attributes. The coating is thus applied to the bar while molten, by permitting the bar to pass through a falling curtain of liquid coating, at the same time passing over a plate or rollers which permit coating to be applied to the under surface of the bar, and excess coating is blown off by means of air jets. Finally, the enrobed bar passes through a cooling tunnel where refrigerated air currents remove heat and cause the coating to solidify.

The solid matrix nutritional embodiments for use herein may also be manufactured through a baked application or heated extrusion to produce solid product forms such as cereals, cookies, crackers, and similar other product forms. One knowledgeable in the nutrition manufacturing arts is able to select one of the many known or otherwise available manufacturing processes to produce the desired final product.

Numerous types of packaging are readily available and known to one practicing the art. Desirable packaging characteristics include: effective protection against impact, light, and heat; ease of opening; and efficient sealing for storage stability.

### Methods of Use

The soy protein compositions of the present disclosure may be administered to an infant during at least about the first 12 months after delivery and to the infant's mother during pregnancy and/or breastfeeding to improve memory and/or cognition in the infant. Specifically, it has been discovered that an improvement in memory and/or cognition in an infant can be achieved by administering soy protein, for example, in the form of a soy protein composition of the present disclosure, to a pregnant woman for at least about three months prior to delivery of the infant and, after delivery, administering soy protein to the infant for at least about 12 months, and in some embodiments until the infant reaches from about 1 year of age to about 5 years of age. For example, the soy protein may be administered to the infant's mother during pregnancy and breastfeeding, and may be administered directly to the infant after delivery by administering a soy protein-containing composition, such as an infant or nutritional formula comprising soy protein, to the infant. By administering soy protein to an infant during early brain development, for example, to the infant's mother during pregnancy and breastfeeding and/or to the infant within the first 12 months following delivery, the memory and/or cognition of the infant is improved.

Thus, in one embodiment, the present disclosure is directed to a method of improving memory in an infant. The method comprises administering to a pregnant woman soy protein for at least about three months prior to delivery of the infant and, after delivery, administering soy protein to the infant for at least about 12 months. An improvement in memory can be, for example, an improvement in hippocampal-dependent tasks such as spatial memory and related forms of learning and memory. An improvement in memory can be measured using any suitable technique known in the art such as, for example, the Morris water maze.

In another embodiment, the present disclosure is directed to a method of improving cognition in an infant. The method comprises administering to a pregnant woman soy protein for at least about three months prior to delivery of the infant and, after delivery, administering soy protein to the infant for at least about 12 months.

The terms "cognition" and "cognitive function" as used herein are interchangeable and refer to a range of functions: intelligence (fluid, crystallised), creativity (generate and open to new ideas, perceive new relationships), memory (encoding, storage, retrieval), executive functions (dealing with novelty, planning & implementation, monitoring performance, vigilance, inhibition of task irrelevant info), cognitive resources (speed of information processing, working memory capacity, attentional capacity), and the like. An improvement in cognition can include an improvement or enhancement of cognitive development. The term "cognitive development" as used herein refers to the development of cognition (cognitive function) over time. The quality of cognition (cognitive function) in a human can be assessed by tests measuring cognitive performance. As cognition (cognitive function) of a growing infant or child develops over time with brain development, measuring the cognitive performance over time for a given infant or child, provides a measure of cognitive development. For example, for children aged 6-9 years, cognitive development is generally recognized by: a spurt in information processing capacity and/or executive functions, and development of the frontal lobes of the brain (involved in executive functions). These growth spurts may also occur at other periods during childhood (e.g. 0-2 years and mid-teens).

The development of the central nervous system is a complex and long-lasting process that starts early in gestation when the neural tube begin to develop. There are several neurological processes that develop more actively during the last trimester of gestation such as axon and dendrite sprouting, synapses formation, glial cell proliferation, and acetylcolinergic systems. Some of these processes continue to develop after birth, more actively during the first year of life and slowly later in life. Myelination occurs postnatally during the first year of life.

As noted above, soy protein is administered to the infant's mother during pregnancy in accordance with the methods of the present disclosure. The soy protein is administered to the pregnant woman for at least about 4 weeks prior to delivery of the infant, preferably for at least about 6 weeks prior to delivery, more preferably for at least about 3 months prior to delivery, preferably at least about one third of the pregnancy, or for at least about 6 months prior to delivery, and more preferably is administered for the duration of the pregnancy. The soy protein may be administered to the pregnant woman in the form of a nutritional composition, such as described herein. The soy protein may be administered to the pregnant woman on any suitable dosing schedule, for example, weekly, daily, and/or multiple times per day. In one embodiment, the soy protein is preferably administered to the pregnant woman on a daily basis. Preferably, the soy protein composition provides the pregnant woman at least about 3 g/day of soy protein, more typically from about 3 g/day to about 54 g/day of soy protein, and more typically from about 8 g/day to about 18 g/day of soy protein.

After delivery, the soy protein is administered to the infant for at least about 3 months, preferably for at least about 6 months, and more preferably, until the infant reaches about 12 months of age. In some embodiments, the soy protein may be administered to the infant until the infant reaches from about 1 year of age to about 5 years of age. The soy protein may be administered to the infant after delivery by administration of a soy protein-containing composition, such as an infant or nutritional formula comprising soy protein. Following delivery, soy protein may also be administered to the infant's mother during lactation and breastfeeding of the infant.

In embodiments where the soy protein is administered to the breastfeeding mother, the breastfeeding woman is administered soy protein, preferably for the duration of breastfeeding, i.e., until weaning. The soy protein may be administered to the breastfeeding woman in the form of a nutritional composition, such as described herein. The soy protein may be administered to the breastfeeding woman on any suitable dosing schedule, for example, weekly, daily, and/or multiple times per day. In one embodiment, the soy protein is preferably administered to the breastfeeding woman on a daily basis. Preferably, the soy protein composition provides the breastfeeding woman at least about 3 g/day of soy protein, more typically from about 3 g/day to about 54 g/day of soy protein, and more typically from about 8 g/day to about 18 g/day of soy protein.

It should be understood that not all women breastfeed their infants, and not all women who breastfeed their infants will breastfeed for at least about 3 months, or at least about 6 months, or for 12 months following delivery. Thus, in some embodiments, the infant is administered soy protein following delivery, for example, by administering the infant an infant and/or nutritional formula comprising soy protein. In one embodiment, an infant formula comprising soy protein is administered to the infant in addition to breastfeeding, for example, as a supplement to breastfeeding and/or after weaning for at least about 3 months, or until the infant reaches at least about 12 months of age. In some embodiments, the soy protein is administered to the infant until the infant reaches from about 1 year of age to about 5 years of age. In this embodiment, a suitable nutritional formulation, such as PediaSure®, may be re-formulated to include soy protein and administered to the child. In another embodiment, the infant is administered an infant and/or nutritional formula comprising soy protein immediately following delivery as the sole source of soy protein (i.e., without prior or concurrent breastfeeding), until the infant reaches at least about 12 months of age, or until the infant reaches from about 1 year of age to about 5 years of age.

The infant will preferably be provided with at least about 4 g/day of soy protein, more typically from about 4 g/day to about 40 g/day of soy protein, and still more typically from about 6 g/day to about 18 g/day of soy protein.

### EXAMPLES

The following examples further describe and demonstrate specific embodiments within the scope of the present disclosure. The examples are given solely for the purpose of illustration and are not to be construed as limitations of the present disclosure, as many variations thereof are possible without departing from the spirit and scope of the disclosure. All exemplified amounts are weight percentages based upon the total weight of the composition, unless otherwise specified.

Each of the exemplified compositions is fed to humans to provide sole, primary, or supplemental nutrition. Each composition contains soy protein, and can be used in a method of the present disclosure to improve memory and/or cognition in an infant.

### Example1

The following example illustrates a nutritional powder embodiment of a soy-based, infant formula that may be administered to an infant in accordance with the methods of the present disclosure. Ingredients for manufacturing 100 kg of the nutritional powder infant formula are listed in Table 3.

**Table 3: Ingredients for a Powdered Infant Formula Containing Soy Protein Isolate**

| **Ingredient** | | **Quantity to formulate 100 kg of nutritional powder** |
|---|---|---|
| Lipid | | 28.0 kg |
| High oleic safflower oil | | (11.5 kg) |
| | Soy bean oil | (8.1 kg) |
| | Coconut oil | (8.4 kg) |
| | Soy protein isolate | 14.5 kg |
| | Carbohydrate | 62.2 kg |
| | Corn syrup | (52.0 kg) |
| | Sucrose solids | (10.2 kg) |
| | | |

| | **Minerals** | |
|---|---|---|
| | Calcium phosphate | 1.8 kg |
| | Sodium chloride | 338 g |
| | Magnesium chloride | 259 g |
| | Potassium citrate | 1.05 kg |
| | Potassium chloride | 191 g |
| | Potassium iodide | 0.1 g |
| | | |

| | **Vitamins** | |
|---|---|---|
| | Water soluble premix with trace minerals | 143 g |
| | Oil soluble premix (ADEK) | 38 g |
| | Methionine | 159 g |
| | Vitamin C | 43.6 g |

The powdered infant formula is prepared by first dispersing the carbohydrate (sucrose solids), and minerals in water with appropriate heat and agitation to form a carbohydrate-mineral slurry. The soy protein isolate is then dispersed in vegetable oil (soy, coconut, high oleic safflower) along with any oil soluble vitamins, emulsifiers, and antioxidants, with appropriate heat and agitation to form a protein-oil slurry. The formed slurries are then blended together with the corn syrup and the resulting pH adjusted to between 6.0 and 7.0 with an appropriate alkaline solution. Final total solid content of the resulting blend is 45-50.5%. The blend is then emulsified at 100-400 psi, subjected to high temperature steam treatment (HTST) at 160-170°F, and then homogenized using a 2-stage homogenization process at pressures of 900-1300 psi/400 psi. Water-soluble materials such as water soluble vitamins, methionine, and trace minerals are then added to the homogenized mixture, which is then subjected to ultra high temperature (UHT) treatment at 220-250°F. The heat treated mixture is then cooled to 160-180°F and spray dried at about 2300 psi at approximately 3 gal/min to yield a finished infant nutritional powder with a moisture content of 2-3% by weight of the powder.

### Example 2

The following example illustrates a ready-to-feed liquid nutritional soy based infant formula that may be administered to an infant in accordance with the methods of the present disclosure. Ingredients for manufacturing 100 kg of ready-to-feed infant formula are listed in Table 4.

**Table 4: Ingredients for Ready-to-Feed Infant Formula Containing Soy Protein Isolate**

| **Ingredients** | **Quantity to formulate 100 kg of ready-to-feed nutritional formula** |
|---|---|
| Lipid | 3.65 kg |
| High oleic safflower oil | (1.4 kg) |
| Soy bean oil | (1.125 kg) |
| Coconut oil | (1.125 kg) |
| Soy protein isolate | 1.9 kg |
| Carbohydrate | 8 kg |
| Corn syrup | (6.6 kg) |
| Sucrose solids | (1.4 kg) |
| | |

| **Minerals** | |
|---|---|
| Potassium citrate | 65 g |
| Potassium chloride | 13.3 g |
| Magnesium chloride | 36.7 g |
| Sodium chloride | 30.7 g |
| Calcium citrate | 165.1 g |
| Calcium phosphate | 114.3 g |
| Potassium phosphate | 98.3 g |
| Potassium iodide | 0.013 g |
| | |

| **Vitamins** | |
|---|---|
| Water soluble premix with trace minerals | 17.6 g |
| Oil soluble premix (ADEK) | 4.89 g |
| Methionine | 20.8 g |
| Vitamin C | 43.6 g |

The ready-to-feed nutritional infant formula is prepared by first dispersing the carbohydrate (sucrose solids), and minerals in water with appropriate heat and agitation to form a carbohydrate-mineral slurry. The soy protein isolate is then dispersed in vegetable oil (soy, coconut, high oleic safflower) along with any oil soluble vitamins, emulsifiers, and antioxidants, with appropriate heat and agitation to form a protein-oil slurry. The formed slurries are then blended together with corn syrup and the resulting pH adjusted to between 6.0 and 7.0 with an appropriate alkaline solution. Final total solid content of the resulting blend is 20-30%. The blend is then emulsified at 100-500 psi, subjected to high temperature steam treatment (HTST) at 143-154°C, and then homogenized using a 2-stage homogenization process at pressures of 3000-4000 psi/500 psi. Water-soluble materials such as water soluble vitamins, methionine, and trace minerals are then added to the homogenized mixture. The mixture is diluted with water to a solid content of 12-13%, packaged and sealed into appropriate containers, and then sterilized at 115-126°C.

### Example 3

The following example illustrates a human milk fortifier powder, which is added to human milk that may be administered to an infant in accordance with the methods of the present disclosure. Ingredients for manufacturing 8,172 kg of powdered human milk fortifier are listed in Table 5.

**Table 5: Bill of Materials for Powdered Human Milk Fortifier Containing Soy Protein**

| **Ingredients** | **Quantity to formulate 8,172 kg of fortifier** |
|---|---|
| Ingredient Water | 16,205 L |
| Corn syrup solids | 1603 kg |
| Magnesium chloride | 96.2 kg |
| Potassium citrate | 223.8 kg |
| Sodium citrate | 6.6 kg |
| Sodium chloride | 15.4 kg |
| MTC oil | 801 kg |
| Lecithin | 16.6 kg |
| Vitamin A | 2.36 kg |
| Vitamin D | 359.3 g |
| Vitamin K | 27.5 g |
| Tocopherol acetate | 15.8 kg |
| Calcium carbonate | 33.1 kg |
| Tricalcium phosphate | 646 kg |
| Soy protein concentrate | 1506 kg |
| Non fat dry milk | 3307 kg |
| Potassium citrate | 257.2 g |
| Ferrous sulfate | 3.7 kg |
| Zinc sulfate | 11.1 kg |
| Copper sulfate | 1.84 kg |
| Manganese sulfate | 0.320 kg |
| Sodium selenate | 0.001 kg |
| Niacinamide | 0.98 kg |
| Riboflavin | 1.14 kg |
| Calcium pantothenate | 4.08 kg |
| Pyridoxine hydrochloride | 0.655 kg |
| m-inositol | 9.55 kg |
| Biotin | 0.0727 kg |
| Folic acid | 0.0775 kg |
| Cyanocobalamin | 0.0016 kg |
| Ascorbic acid | 153.5 kg |

A carbohydrate/mineral slurry is prepared by heating 2,763 liters of ingredient water to 54°C-62°C. With agitation, the specified amounts of corn syrup solids (Maltrin M200 distributed by Grain Processing Corporation, Muscatine, Iowa), magnesium chloride, sodium chloride, sodium citrate, potassium citrate, ultra micronized tricalcium phosphate and calcium carbonate are added to the heated water. The slurry is held under agitation at 54°C-62°C for not longer than six hours until it is blended with the other slurries.

An oil blend is prepared by heating the specified amount of MCT oil (distributed by Stepan, Maywood, N.J.) to 32°C-37°C with agitation. An emulsifier (standard fluid lecithin distributed by Central Soya, Ft. Wayne, Ind.) is then added under agitation and allowed to dissolve. Vitamin A,D,K and Vitamin E (distributed by Vitamins, Inc., Chicago, I11.) are then added to the slurry with agitation. The completed oil slurry is held under moderate agitation at a temperature from 26°C to 48°C for a period of no longer than six hours until it is blended with the other slurries.

A protein-in-water slurry is prepared by heating 9,053 liters of ingredient water to 48°C-60°C. With agitation, the specified amount of soy protein concentrate and nonfat dry milk is added to the heated water. The completed protein-in-water slurry is not held but blended directly with the other slurries.

The protein-in-water, oil blend and carbohydrate/mineral slurries are blended together with agitation and the resultant blend is maintained at a temperature from 51°C to 60°C. After waiting for at least five minutes with agitation the final blend pH is adjusted with 1N KOH to a pH from 6.45 to 6.80. The total solids content of the final blend is 30%. The final blend is held for no longer than two hours after the pH check.

After waiting for a period of not less than five minutes nor greater than two hours, the blend is subjected to deaeration, high-temperature-short-time heat treatment, and homogenization, as follows: deaerate the blend at 10-15 inches Hg; emulsify the blend at 900-1100 psig in a single stage homogenizer; pass the blend through a plate/coil heater and heat the mix to 71 °C to 82°C; homogenize the blend at 3900 to 4100/400 to 600 psig in a double stage homogenizer; pass the blend through a 16 second hold tube at a temperature from 73°C to 85°C; cool the blend to a temperature from 1°C to 7°C; and store the blend at a temperature from 1°C to 7°C.

After the above steps have been completed, appropriate analytical testing for quality control is conducted. Based on the analytical results of the quality control tests, batch corrections are made if need be. Final blend total solids are from 29% to 31 %.

A water soluble vitamin solution, ascorbic acid solution and trace mineral solution are prepared separately and added to the processed blend.

The ascorbic acid solution is prepared by adding the required amount of ascorbic acid to 2,453 liters of 10°C to 37°C water with agitation.

The mineral solution is prepared by heating 321 liters of ingredient water to 37°C to 65°C. Under agitation, add the required amount of potassium citrate and ferrous sulfate. Allow to agitate until the solution is a clear green color. Add the required amounts of zinc sulfate, copper sulfate, manganese sulfate and sodium selenate to the green mineral solution. Agitate five minutes minimum.

The water soluble vitamin solution is prepared by heating 530 liters of ingredient water to 37°C to 65°C. The required quantities of niacinamide, riboflavin, calcium pantothenate, pyridoxine hydrochloride, thiamine hydrochloride, m-insitol, biotin, folic acid and cyanocobalamin are added to the heated water.

All of the ascorbic acid solution, the mineral solution and water soluble vitamin solution is then added to the blended slurry under agitation.

The final mix is preheated through a plate heater to 71 °C-82°C before going to a surge tank. The mix leaves the surge tank and passes through the steam injector where it is heated to 88°C-93°C. The mix enters the vapor-flash chamber where it is cooled to 71 °C-82°C, then pumped through an in-line 200 micro filter prior to the high pressure pump and into the dryer. The dryer settings are as follows: the nozzle pressure is at 3000-5000 psig, the liquid flow rate is at 11 gpm max, the in-going air temperature is at 160°C-207°C, and the outgoing air temperature is at 82°C-108°C.

To control bulk density, dispersibility, particle size, moisture and physical stability, the specific spray nozzle, nozzle pressure, drying temperatures and fine reinjection parameters may vary depending upon the drying conditions of the day. The powder passes from the dryer into the powder cooler where the powder is cooled to below 43°C. The cooled powder is stored in appropriate containers until being filled in individual packets.

### Example 4

The following example illustrates a vanilla flavored liquid nutritional product containing soy protein that may be administered to a pregnant or breastfeeding woman in accordance with the methods of the present disclosure. Ingredients for manufacturing 454 kg (1,000 lbs) of the nutritional product are listed in Table 6.

**Table 6: Bill of Materials for Liquid Vanilla Flavored Product Containing Soy Protein Isolate**

| **Ingredient** | **Quantity per 454 kg** | **Quantity (lbs) per 1000 lbs** |
|---|---|---|
| Water | 347 kg | 765 |
| Maltodextrin | 35.6 kg | 78.5 |
| Sucrose | 32.2 kg | 70.9 |
| Soy protein isolate | 28 kg | 61.7 |
| High oleic safflower oil | 1.76 kg | 3.87 |
| Canola oil | 1.76 kg | 3.87 |
| Potassium citrate | 1.77 kg | 3.90 |
| Magnesium phosphate | 1.27 kg | 2.80 |
| Vanilla flavor | 0.91 kg | 2.00 |
| Cellulose gel | 0.91 kg | 2.00 |
| Corn oil | 0.88 kg | 1.94 |
| Calcium citrate | 0.45 kg | 1.00 |
| Choline chloride | 240 gm | 0.529 |
| Sodium citrate | 227 gm | 0.500 |
| Soy lecithin | 197 gm | 0.435 |
| UTM/TM premix | 163 gm | 0.359 |
| Ascorbic acid | 148 gm | 0.326 |
| Carrageenan | 68 gm | 0.150 |
| Vitamin DEK premix | 44 gm | 0.0970 |
| WSV Premix | 29 gm | 0.0639 |
| 30% Beta carotene | 3.7 gm | 0.00816 |
| Vitamin A Palmitate Premix | 1.6 gm | 0.00364 |
| Potassium Iodide | 233 mg | 0.000515 |

The liquid nutritional product is manufactured by preparing three slurries which are blended together, heat treated, standardized, packaged and sterilized.

Specifically, a carbohydrate/mineral slurry is prepared by combining the specified amount of cellulose gel (Avicel CL-611 distributed by FMC Corp., Philadelphia, Pa.) with the required amount of water under high agitation. The mixture is heated to a temperature of from about 140°F to about 150°F with agitation. The cellulose gel is dissolved before the mineral addition. The following minerals are then added in the order listed, under high agitation: potassium citrate, sodium citrate, and UTM/TM premix (U.S. Pat. No. 5,221,545 teaches how to produce the premix used in this example). The slurry is held under agitation for a minimum of 5 minutes. The remaining minerals are added, in the order listed under high agitation: potassium iodide, and magnesium phosphate. Next, the maltodextrin (Lodex 15 distributed by American Maize, Hammond, Ind.) is added to the slurry under high agitation, and is allowed to dissolve. The sugar (sucrose) is then added under high agitation and allowed to dissolve. The completed carbohydrate/mineral slurry is held under high agitation at a temperature from about 140°F to about 150°F for not longer than twelve hours until it is blended with the other slurries.

An oil blend is prepared by combining and heating the high oleic safflower oil, corn and canola oil to a temperature from about 130°F to about 140°F with agitation. An emulsifier (soy lecithin) is then added under agitation and allowed to dissolve. The Vitamin D,E,K premix (distributed by Vitamin, Inc., Chicago, I11.), Vitamin A Palmitate, and 30% beta-carotene are then added to the slurry with agitation. A stabilizer (carrageenan Viscarin SD-389® distributed by FMC Corp., Philadelphia, Pa.) is added to the slurry with agitation and allowed to disperse. The completed oil slurry is held under moderate agitation at a temperature from about 130°F to about 140°F for a period of no longer than twelve hours until it is blended with the other slurries.

A 10 to 12% protein-in-water slurry is prepared by first adding the soy protein isolate (Soy protein FXP-E-0101 distributed by Protein Technologies International, St. Louis, Mo.) to the required amount of water under high agitation. The slurry is heated to a temperature from about 85°F to about 95°F with agitation and held for a minimum of 15 minutes. The slurry temperature is then increased to a temperature of about 115°F to about 125°F and held for a minimum of 5 minutes. The slurry temperature is then increased to a temperature of about 145°F to about 155°F. Cocoa powder (8 lbs.) may be dispersed in the protein slurry for a chocolate flavored product. The completed protein-in-water slurry is held under moderate agitation at a temperature from about 145°F to about 155°F for a period of no longer than two hours until it is blended with the other slurries.

The protein-in-water and oil slurries are blended together with agitation and the resultant blend is maintained at a temperature from about 140°F to about 150°F. After waiting for at least five minutes, the carbohydrate/mineral slurry is added to the blend from the preceding step with agitation and the resultant blend is maintained at a temperature from about 140°F to about 150°F. The total solids content of the final blend is about 25%. The blend pH is brought up to about 6.8 to about 7.0 with 1N KOH.

After waiting for a period of not less than five minutes nor greater than two hours, the blend is subjected to deaeration, ultra-high-temperature treatment, and homogenization, as follows: deaerate the blend at 10-15 in. Hg; preheat the blend through a plate/coil heat exchanger to 155-165°F.; emulsify the blend at 900-1100 psig; pass the blend through a plate/coil heater and heat the mix to from about 250°F to about 255°F; ultra high temperature heat the blend to a temperature of about 298°F to about 302°F with a hold time of about 5 seconds; reduce the temperature of the blend to from about 250°F to about 255°F by passing it through a flash cooler; reduce the temperature of the blend to from about 160°F to about 170°F by passing it through a plate/coil heat exchanger; homogenize the blend at about 3900 to about 4100/about 400 to about 600 psig; pass the blend through a hold tube for at least 16 seconds at temperature from about 165°F to about 175°F; cool the blend to a temperature from about 340°F to about 45°F by passing it through a heat exchanger; and store the blend at a temperature from about 34°F to about 45°F with agitation.

After the above steps have been completed, appropriate analytical testing for quality control is conducted. Based on the analytical results of the quality control tests, an appropriate amount of water is added to the batch with agitation for dilution to about 22% total solids.

A vitamin solution and a flavor solution are prepared separately and added to the processed blend.

The vitamin solution is prepared by adding the following ingredients to the required amount of water, under agitation: Ascorbic Acid, Water Soluble Vitamin Premix (distributed by Fortitech, Inc., Schenectady, N.Y.) and Choline Chloride. The vitamin solution pH is adjusted to from about 6 to about 10 with 45% KOH. The vitamin slurry is then added to the blended slurry under agitation.

The flavor solution is prepared by adding the natural and artificial flavor to the appropriate amount of water with agitation. The flavor slurry is then added to the blended slurry under agitation.

The product pH is adjusted to achieve optimal product stability. The completed product is then placed in suitable containers and subjected to terminal sterilization.

### Example 5

The following example illustrates an orange and carrot juice soy beverage that may be administered to a pregnant or breastfeeding woman in accordance with the methods of the present disclosure. Ingredients for manufacturing 6.8 kg of the beverage are listed in Table 7.

**Table 7: Bill of Materials for Orange and Carrot Juice Soy Product**

| **Ingredients** | **Quantity** |
|---|---|
| Water | 5.2 kg |
| Soy protein | 332 gm |
| Orange juice (65 Brix) | 309 gm |
| Carrot juice (70 Brix) | 194.4 gm |
| Pectin | 18 gm |
| Cane juice solids | 488 gm |
| Canola oil | 3.35 gm |
| Ascorbic acid | 2.22 gm |
| Vitamin DEK premix* | 0.66 gm |
| WSV premix** | 0.44 gm |
| Vitamin A palmitate | 0.07 gm |

| | |
|---|---|
| *per gm of Vitamin DEK premix: 5815 IU vitamin D₃, 671 IU vitamin E, 1.12 IU vitamin K₁. **per gm of WSV premix: 375 mg niacinamide, 242 mg calcium pantothenate, 8.4 mg folic acid, 62 mg thiamine chloride, 48 mg riboflavin, 59 mg pyridoxine hydrochloride, 165 mcg cyanocobalamin, and 7305 mcg biotin. | |

The beverage is manufactured by preparing three slurries which are blended together, heat treated, standardized, and aseptically packaged. Specifically, a carbohydrate/mineral slurry is prepared by combining the specified amount of pectin with the required amount of water under high agitation. The mixture is heated to a temperature of from about 140°F to about 150°F with agitation. The slurry is held under agitation for a minimum of 5 minutes. The cane juice solids is added under high agitation and allowed to dissolve. The completed carbohydrate/mineral slurry is held under high agitation at a temperature from about 140°F to about 150°F for not longer than twelve hours until it is blended with the other slurries.

An oil blend is prepared by combining and heating the canola oil to a temperature from about 130°F to about 140°F with agitation. The Vitamin D,E,K premix (distributed by Vitamin, Inc., Chicago, III.) and Vitamin A Palmitate are then added to the slurry with agitation. The completed oil slurry is held under moderate agitation at a temperature from about 130°F to about 140°F for a period of no longer than twelve hours until it is blended with the other slurries.

A 10 to 12% protein-in-water slurry is prepared by first adding the soy protein (Soy protein distributed by Protein Technologies International, St. Louis, Mo.) to the required amount of water under high agitation. The slurry is heated to a temperature from about 85°F to about 95°F with agitation and held for a minimum of 15 minutes. The required amount of orange juice concentrate and carrot juice is added under agitation. The completed protein-in-water slurry is held under moderate agitation at a temperature from about 145°F to about 155°F for a period of no longer than two hours until it is blended with the other slurries.

The protein-in-water and oil slurries are blended together with agitation and the resultant blend is maintained at a temperature from about 140°F to about 150°F. After waiting for at least five minutes, the carbohydrate slurry is added to the blend from the preceding step with agitation and the resultant blend is maintained at a temperature from about 140°F to about 150°F.

After waiting for a period of not less than five minutes nor greater than two hours, the blend is deaerated at 10-15 in. Hg, preheated through a plate/coil heat exchanger to 155-165°F, emulsified at 900-1100 psig, passed through a plate/coil heater and heated to from about 250°F to about 255°F, heated to a temperature of about 298°F to about 302°F with a hold time of about 5 seconds, temperature reduced to from about 250°F to about 255°F by passing through a flash cooler, temperature reduced to from about 160°F to about 170°F by passing through a plate/coil heat exchanger, homogenized at about 3900 to about 4100/about 400 to about 600 psig, passed through a hold tube for at least 16 seconds at temperature from about 165°F to about 175°F, cooled to a temperature from about 34°F to about 45°F by passing through a heat exchanger, and stored at a temperature from about 34°F to about 45°F with agitation.

After the above steps have been completed, appropriate analytical testing for quality control is conducted. Based on the analytical results of the quality control tests, an appropriate amount of water is added to the batch with agitation for dilution to the preferred total solids.

A vitamin solution is prepared separately and added to the processed blend.

The vitamin solution is prepared by adding the following ingredients to the required amount of water, under agitation: Ascorbic Acid and Water Soluble Vitamin Premix (distributed by Fortitech, Inc., Schenectady, N.Y.). The vitamin solution pH is adjusted to from about 6 to about 10 with 45% KOH. The vitamin slurry is then added to the blended slurry under agitation.

The final blend pH is adjusted to 4.0 with 10% malice and 10% citric acid solutions. The completed product is then aseptically filled in to suitable containers.

### Example 6

The following example illustrates a lemon flavored nutritional bar that may be administered to a pregnant or breastfeeding woman in accordance with the methods of the present disclosure. Ingredients for manufacturing a 1 kg bar are listed in Table 8.

**Table 8: Bill of Materials for Lemon Flavored Nutritional Bar Containing Soy Protein**

| **Ingredients** | **Quantity per 1 kg** |
|---|---|
| Sugar free white confectionery coating | 190 gm |
| Soy protein | 162.3 gm |
| Honey | 143.5 gm |
| Fibersol 2(E) | 107.9 gm |
| High fructose corn syrup | 91.5 gm |
| Rice crisps | 63.5 gm |
| Soy polysaccharide | 49.1 gm |
| Glycerin | 46.3 gm |
| Vitamin/mineral premix* | 28.2 gm |
| High oleic safflower | 27.9 gm |
| Fructooligosaccharide | 21.2 gm |
| Encapsulated guar gum | 16.5 gm |
| Microcrystalline cellulose | 13.3 gm |
| Canola | 11.7 gm |
| NA lemon crème | 10.1 gm |
| Fructose | 7.8 gm |
| Citric acid | 3.3 gm |
| m-inositol | 2.9 gm |
| Soy lecithin | 2.8 gm |
| α-Tocopherol acetate | 911 mg |

| | |
|---|---|
| *Vitamin/mineral premix (per g premix): 1219 IU vitamin A, 90 IU vitamin D₃, 38 IU vitamin E (α-Tocopherol acetate), 19.8 mcg vitamin K₁, 90 mg vitamin C, 4.9 mg niacinamide, 2.33 mg calcium pantothenate, 0.187 mg folic acid, 0.377 mg thiamine mononitrate, 0.645 mg riboflavin, 0.457 mg pyridoxine hydrochloride, 2.91 mcg cyanocobalamin, 74.65 mg biotin, 2.77 mg zinc, 3.3 mg iron, 0.797 mg manganese, 0.33 mg copper, 35.1 mcg iodine, 35.9 mcg chromium, 24.3 mcg molybdenum, 24.6 mcg selenate, 227 mg calcium, 56.5 gm magnesium, and 120.3 mg phosphorous. | |

The bar core is prepared by mixing a dry preblend in two stages, adding the liquid preblend followed by the canola oil. A dry preblend is prepared by mixing the required amounts of soy protein (distributed by Protein Technologies International, St. Louis, Mo.), fructooligosaccharide (distributed by Golden Technologies Company of Golden, Colo.), m-inositol and soy polysaccharide (distributed by Protein Technologies International, Louis, Mo. under the name of Fibrim 1450) for 10 minutes in a Ribbon Blender. The required amounts of the following ingredients are added to the Ribbon Blender: microcrystalline cellulose, citric acid and Fibersol 2(E) (distributed by Matsutani Chemical Industry Co., Hyogo, Japan) and mixed for an additional 20 minutes. 25 kg of the blend is removed from the bottom of the blender and added back through the top of blender and mixed for an additional 5 minutes. The dry preblend is stored in drums.

The required amount of the dry preblend above is added to a Dough Mixer along with the required amounts of lemon creme flavor (distributed by Givaudan Roure, Cincinnati, Ohio under the name of NA Lemon Creme), vitamin/mineral premix (distributed by Fortitech, Schenectady, N.Y.), α-tocopherol acetate, microencapsulated guar gum (distributed by Coating Place, Verona, Wis.) and rice crisp (distributed by Weetabix, Clinton, Mass. under the name of Densifeid Crisp Rice-No sugar, salt or malt) and mixed for 200 strokes by the Dough Mixer.

The liquid preblend is prepared by adding the required amount of high fructose corn syrup (distributed by ADM Corn Processing, location under the name of cornsweet 95), honey, glycerine, crystalline fructose, high oleic safflower oil (distributed by California Oils, Richmond, Calif.) and bleached lecithin in a Hobart Mixer for 5 minutes.

All of the liquid preblend is added to the dry preblend in the Dough Mixer within the first 60 strokes. The total mixing time is set for 500 strokes. When 100 to 150 strokes is remaining, the required amount of canola oil is added to the Dough Mixer.

The mixed material is transferred from the Dough Mixer to the extruder. The extruder is adjusted to produce bar cores from 33.3 gm to 37.3 gm, the target weight is set at 35.3 gm. The extruded bar cores are conveyed through the cooling tunnel set at the target range of 8 to 12°C.

The bar cores are coated with a maltitol white confectionery coating prepared as follows. The coating is melted at a temperature range between 46-48°C. The temperature is not allowed to exceed 50°C in the melter. Once melted, the coating is held between 46-48°C for 30 minutes to insure destruction of all unstable crystal.

The extruded bar cores are conveyed through the enrober. The coating is applied to the bars at a temperature range between 41-48°C. The coating temperature is not allowed to exceed 50°C in the enrober. The target percent coating is 16.3%, 19% is the maximum coating percent.

After the enrober, the coated bars are conveyed through the cooling tunnel set at 10°C, the cooling range is from 8-13°C. The bars pass through a metal detector prior to wrapping. The cooled bars are packaged in foil wrap.

### Example 7

The effect of administering soy protein to pregnant and breastfeeding women on memory and cognition of offspring was tested using an animal model.

### Subjects

Pregnant Sprague Dawley (SD) rats (n=6) were allowed to consume ad libitum either a diet containing soy protein (17.5 g soy protein per 100 g feed) as the only source of protein or a similar diet containing casein as the sole protein source (control) starting from the third week of gestation to weaning.

After birth, a total number of 18 male and female rat pups were assigned to one of 2 groups, depending on the diet their mother received: Group 1 (n=9, 3 females and 6 males) (soy protein) or Group 2 (n=9, 2 females and 7 males) (control). The Group 1 rats were reared by dams administered the soy protein diet, and the Group 2 rats were reared by dams administered the control diet containing casein. The rats were weaned beginning at postnatal day 21 (PN21). Behavioral assessments were conducted from postnatal day 27 (PN27) to postnatal day 35 (PN35).

Food and water were available ad libitum in the home cage throughout the behavioral assessment. The Group 1 rats received the same soy protein diet administered to their mothers, and the Group 2 (control) rats were administered the control diet containing casein. The animals were maintained on a 12:12 hour light-dark cycle, with the behavioral assessments being performed during the light phase of the cycle. All the experimental procedures were approved by the University of Granada Ethics Committee, and were in accordance with the European Communities Council Directive of November 24, 1986 (86/609/EEC).

### Morris hidden platform task (PN27-PN35)

The Morris hidden platform task was performed on postnatal days 27-35. The pool used in this task consisted of a 150 cm diameter and 50 cm deep circular plastic tank with a removable visible circular platform of 11 cm diameter circular platform. The temperature of the water was maintained at 24-26°C. The pool was divided conceptually into four quadrants, and the platform was placed approximately 35 cm from the pool border in the center of one of the quadrants. Water was stained with non-toxic black ink so the computerized system used to collect data could detect the white rat.

Training consisted of one daily block. Each block consisted of four trials separated by a 5 minute inter-trial interval. Each trial began by placing the subject into the water facing the pool wall at one of four compass conditions (east, west, north, or south). Each subject was released once from each of the four compass points during a block of four trials. The order varied randomly. The animal was allowed to swim freely for 60 seconds or until it climbed onto the platform. If it did not find the platform within 60 seconds, it was placed there by the experimenter. All the rats spent the last 15 seconds of each trial on the platform. During the intertrial intervals, the subjects were group-housed in a cage lined with an electric heating pad and covered to block the view of the swimming pool and the room. On the probe trial at the end of training, the subject was allowed to swim freely for 60 seconds after being released from a fixed starting location.

A video system and associated software allowed several dependent variables to be recorded: escape latency, path length, speed, time spent in the target quadrant, proximal area, platform area crossings, and tigmotaxis.

Each subject received 6 blocks of training, applied in one daily session during 6 consecutive days and several probe trials without platform in order to assess spatial memory 24 and 48 hours after the last training block.

### Results

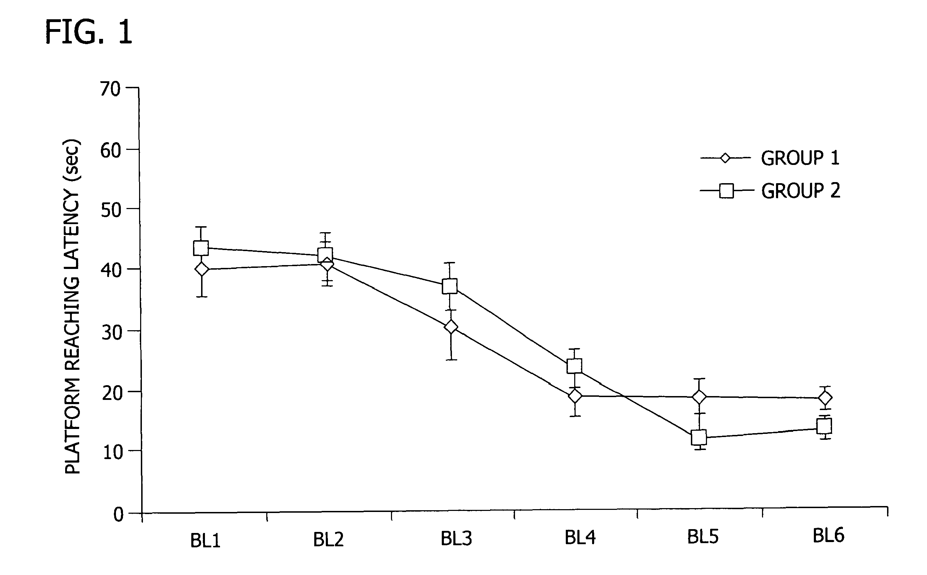

Platform reaching latency: Figure 1 shows the mean (±set) latency to reach the hidden platform by the Group 1 and 2 rats during each of the 6 training blocks. Both groups showed spatial learning, i.e., reduced latencies and distances to reach the hidden platform along the training procedure.

A global 2 x 6 (Group x Block) mixed ANOVA analysis, with treated Group as between-group factor and Block as within-subject factor, revealed significant effects of the main factors of Block (F_{(4.64)} = 22.11; p<0.01), but did not reveal significant effects of Group (F_{(1.6)} = 0.21; p>0.6) or the interaction Group x Block (F₍₄.₆₄₎ = 0.83; p>0.5). Post hoc analysis by LSD tests showed that the latencies significantly and progressively decrease from block 3 in both groups, thus reflecting spatial learning. Thus, mean latencies in block 3 were shorter than those recorded in the first two blocks (p<0.06) and no differences were seen between the blocks 5 and 6 (p>0.8).

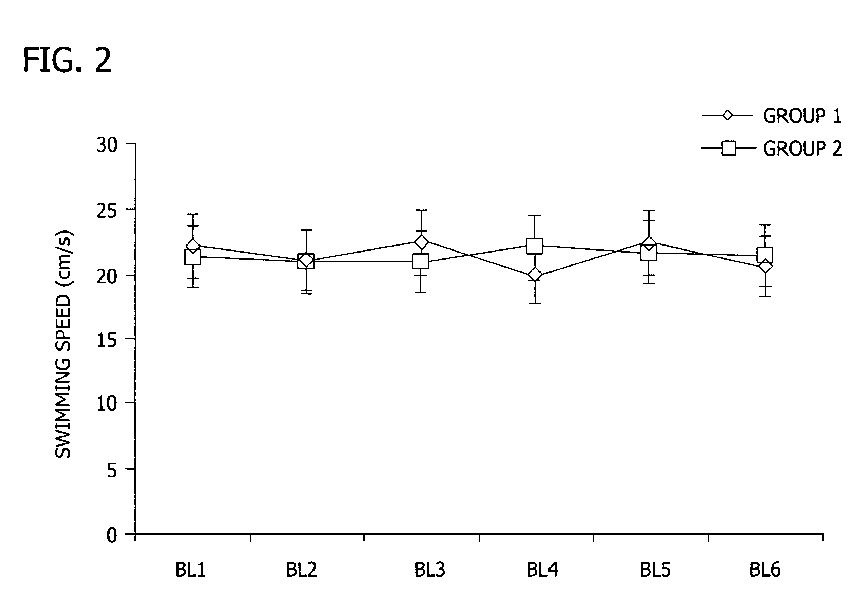

Swimming speed: Figure 2 shows the mean (±SEM) swimming speed of the Group 1 and 2 rats along the blocks of trials during training in the hidden platform task. A 2 x 6 (Group x Block) mixed ANOVA analysis, with treated Group as between-group factor and Block as within-subject factor, revealed no significant effects of the main factors Group (F_{(1.6)} = 0.007; p>0.9), Block (F₍₅.₈₀₎ = 0.29; p>0.9), or the interaction Group x Block (F₍₅.₈₀₎ = 0.62; p>0.6).

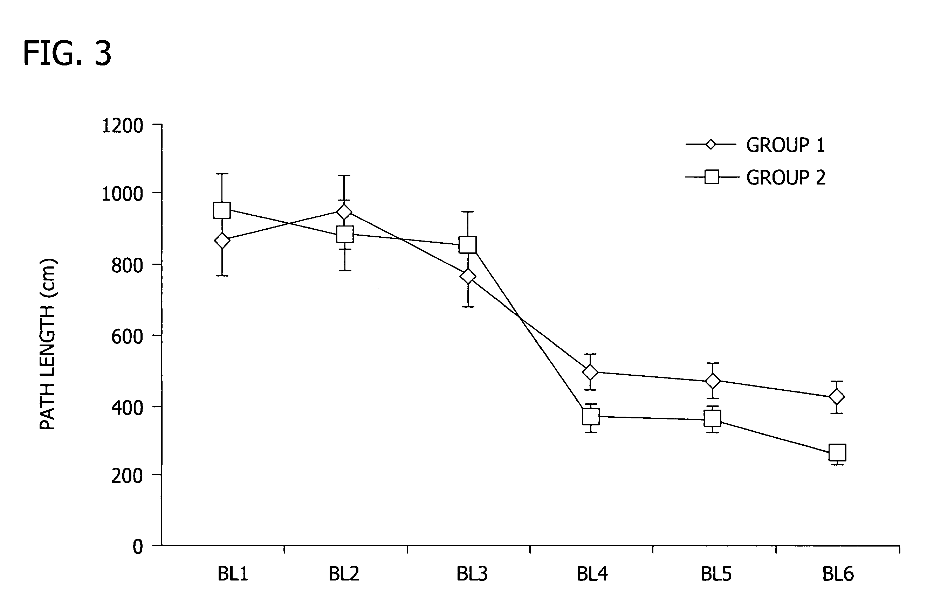

Path length: Figure 3 shows the mean (±SEM) path length of the Group 1 and 2 rats along the blocks of trials during training in the hidden platform task. A 2 x 6 (Group x Block) mixed ANOVA analysis, with treated Group as between-group factor and Block as within-subject factor, revealed significant effects of the main factor Block (F_{(5.80)} = 22.06; p<0.01), but no significant effects of Group (F_{(1.6)} = 1.35; p>0.2), or the interaction Group x Block (F₍₅.₈₀₎ = 0.92; p>0.4). Post hoc analysis by LSD tests showed that the distances significantly and progressively decrease from block 4 in both groups, thus reflecting spatial learning. Thus, mean distances in block 4 were shorter than those recorded in the first blocks (p<0.01) and no differences were seen between the blocks 5 and 6 (p>0.3).

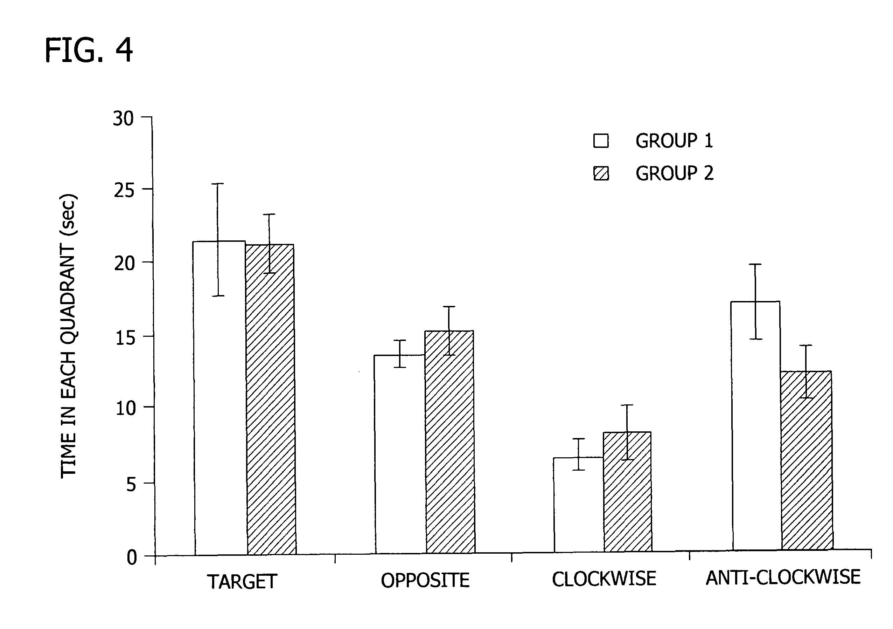

Probe trial (24 hours)―Time in each quadrant during test: Figure 4 shows the time spent in each of the quadrants during the probe trial (24 hours after training) by rats in each group. A 2 x 6 (Group x Quadrants) mixed ANOVA analysis, with treated Group as between-group factor and Quadrant as within-subject factor, did not reveal significant effects of the main factor Group (F_{(1.6)} = 0.36; p>0.5), or the interaction Group x Quadrant (F₍₅.₈₀₎ = 0.74; p>0.5), but it showed a significant effect of Quadrants (F_{(3.48)}= 10.50; p<0.01). Post hoc analysis by LSD tests showed that all groups spent more time in target, opposite and anti-clockwise quadrants (p<0.05) than the clockwise quadrant (p>0.8).

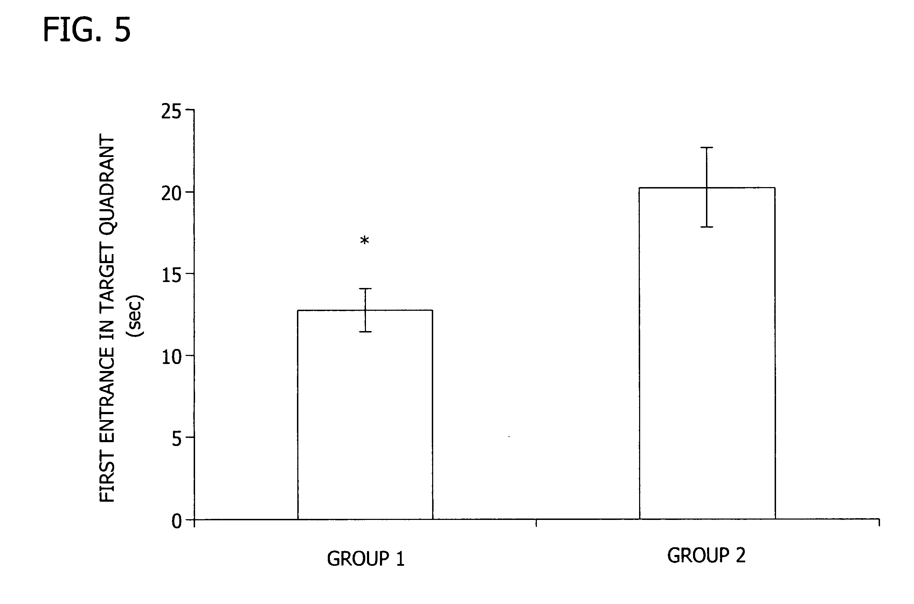

Probe trial (24 hours)―First entrance in target quadrant latency during test: Figure 5 shows the mean (±SEM) first entrance latencies in the quadrant where the platform was located during the test. A better memory of the platform location in Group 1 is supported by the reduced latency to reach the target quadrant during the probe trial. An ANOVA analysis of the latency of the first entrance in the target quadrant during the probe trial revealed a significant effect of Group (F_{(1.6)} = 6.42; p<0.05).

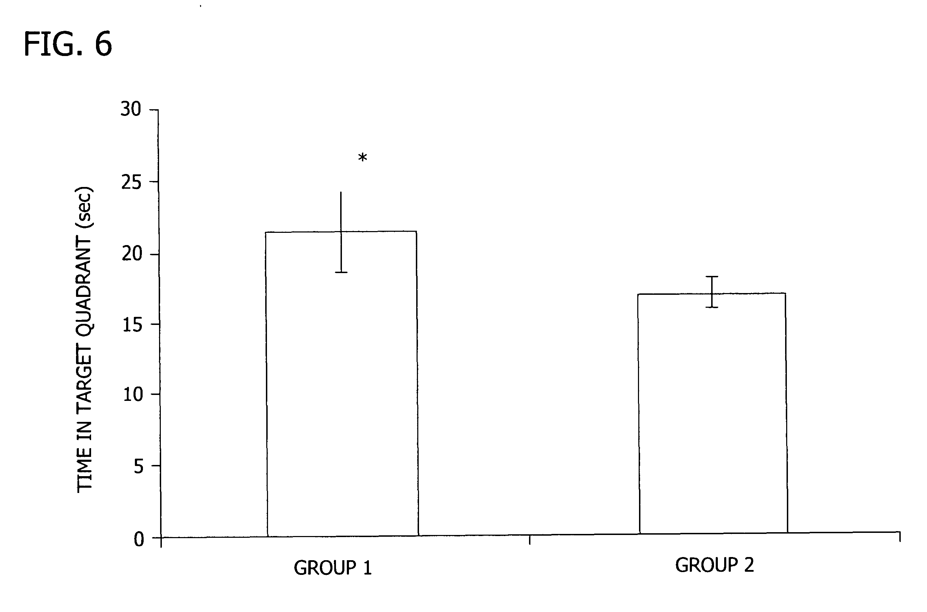

Probe trial (48 hours)―Time in target quadrant during test: Figure 6 shows the time spent in the target quadrant during the probe trial (48 hours after training). An ANOVA analysis of the time in target quadrant during the probe trial revealed a marginal effect of Group (F_{(1.6)} = 4.16; p=0.05).

### Conclusion

The above experiment demonstrated the selective enhancement of spatial memory (Morris water maze—hidden platform task) in developing rats in the soy protein group (Group 1). Both Groups 1 and 2 rats were able to learn the hidden platform water maze task. However, the rats receiving the soy protein supplemented diet (Group 1) seemed to exhibit a better memory of the platform location, since they reached the target quadrant during the probe trial (24 hours) before the Group 2 rats. Consistently, the Group 1 rats spent more time in the target quadrant during the probe trial (48 hours after training) than the Group 2 rats.

## Claims

1. A method of improving memory in an infant, the method comprising:
administering to a pregnant woman soy protein for at least about three months prior to delivery of the infant; and
after delivery, administering soy protein to the infant for at least about 4 months.

2. The method of claim 1 wherein the woman is administered soy protein for at least about six months prior to delivery of the infant.

3. The method of claim 1 wherein the woman is administered soy protein daily during pregnancy.

4. The method of claim 1 wherein the woman is administered from about 3 g/day to about 54 g/day of soy protein during pregnancy.

5. The method of claim 1 wherein soy protein is administered to the woman in a nutritional composition.

6. The method of claim 5 wherein the nutritional composition is selected from the group consisting of a ready-to-feed liquid, a beverage, and a solid matrix nutritional.

7. The method of claim 1 wherein after delivery, the soy protein is administered to the woman during breastfeeding.

8. The method of claim 7, wherein the woman is administered from about 3 g/day to about 54 g/day of soy protein during breastfeeding.

9. The method of claim 7, wherein the woman is administered soy protein until weaning.

10. The method of claim 7 wherein after weaning, the infant is administered soy protein in an infant formula.

11. The method of claim 10 wherein the infant formula provides the infant with from about 4 g/day to about 40 g/day of soy protein.

12. The method of claim 7 wherein after weaning, the infant is administered soy protein in a nutritional formula until the infant reaches from about 1 year of age to about 5 years of age.

13. The method of claim 1 wherein after delivery, the infant is administered soy protein in a nutritional formula.

14. The method of claim 13 wherein the nutritional formula is administered to the infant until the infant reaches from about 1 year of age to about 5 years of age.

15. The method of claim 13 wherein the nutritional formula provides the infant with from about 4 g/day to about 40 g/day of soy protein.

16. The method of claim 1 wherein the memory improvement is an improvement in spatial memory.

17. A method of improving memory in an infant, the method comprising:
administering to a pregnant woman soy protein for at least about three months prior to delivery of the infant;
after delivery, administering soy protein to the woman during breastfeeding of the infant until weaning; and
after weaning, administering an infant formula comprising soy protein to the infant until the infant has reached at least about 12 months of age.

18. A method of improving cognition in an infant, the method comprising:
administering to a pregnant woman soy protein for at least about three months prior to delivery of the infant; and
after delivery, administering soy protein to the infant for at least about 12 months.

19. The method of claim 18 wherein after delivery the soy protein is administered to the woman during breastfeeding.

20. The method of claim 18 wherein after delivery, the infant is administered soy protein in an infant formula.

## Amended claims

### Amended claims in accordance with Rule 137(2) EPC.

**1.** A method of improving memory in an infant, the method comprising:
administering to a pregnant woman soy protein for at least about three months prior to delivery of the infant; and
after delivery, administering soy protein to the infant for at least about 4 months.

**2.** The method of claim 1 wherein the woman is administered soy protein for at least about six months prior to delivery of the infant.

**3.** The method of claim 1 wherein the woman is administered soy protein daily during pregnancy.

**4.** The method of claim 1 wherein the woman is administered from about 3 g/day to about 54 g/day of soy protein during pregnancy.

**5.** The method of claim 1 wherein soy protein is administered to the woman in a nutritional composition.

**6.** The method of claim 5 wherein the nutritional composition is selected from the group consisting of a ready-to-feed liquid, a beverage, and a solid matrix nutritional.

**7.** The method of claim 1 wherein after delivery, the soy protein is administered to the woman during breastfeeding.

**8.** The method of claim 7, wherein the woman is administered from about 3 g/day to about 54 g/day of soy protein during breastfeeding.

**9.** The method of claim 7, wherein the woman is administered soy protein until weaning.

**10.** The method of claim 7 wherein after weaning, the infant is administered soy protein in an infant formula.

**11.** The method of claim 10 wherein the infant formula provides the infant with from about 4 g/day to about 40 g/day of soy protein.

**12.** The method of claim 7 wherein after weaning, the infant is administered soy protein in a nutritional formula until the infant reaches from about 1 year of age to about 5 years of age.

**13.** The method of claim 1 wherein after delivery, the infant is administered soy protein in a nutritional formula.

**14.** The method of claim 13 wherein the nutritional formula is administered to the infant until the infant reaches from about 1 year of age to about 5 years of age.

**15.** The method of claim 13 wherein the nutritional formula provides the infant with from about 4 g/day to about 40 g/day of soy protein.

**16.** The method of claim 1 wherein the memory improvement is an improvement in spatial memory.

**17.** A method of improving memory in an infant according to claim 1, **characterized in that** after delivery, the method comprises administering soy protein to the woman during breastfeeding of the infant until weaning; and
after weaning, administering an infant formula comprising soy protein to the infant until the infant has reached at least about 12 months of age.

**18.** A method of improving cognition in an infant, the method comprising:
administering to a pregnant woman soy protein for at least about three months prior to delivery of the infant; and
after delivery, administering soy protein to the infant for at least about 12 months.

**19.** The method of claim 18 wherein after delivery the soy protein is administered to the woman during breastfeeding.

**20.** The method of claim 18 wherein after delivery, the infant is administered soy protein in an infant formula.

**21.** A method of improving cognition in an infant, the method comprising administering to a pregnant woman soy protein; and after delivery, administering soy protein to the infant.

**22.** A method of improving cognition in an infant, the method comprising administering to a pregnant woman soy protein and after delivery, administering soy protein to the woman during breastfeeding of the infant until weaning.

**23.** A method of improving cognition in an infant, the method comprising administering to a pregnant woman soy protein, and after delivery administering soy protein to the woman during breastfeeding of the infant until weaning, and administering after weaning soy protein to the infant.
